# EUROPEAN PATENT APPLICATION

(11) **EP 4 678 729 A1**
(43) Date of publication of application: **14.01.2026**
(21) Application number: 24382757.3
(22) Date of filing: 12.07.2024
(51) Int. Cl.: C12M 3/06, C12N 5/077

(54) **OSTEOCHONDRAL TISSUE ON A CHIP SYSTEM AND METHOD FOR CULTURING OSTEOCHONDRAL TISSUE**

(71) Applicant: Fundación Para La Investigación Biomédica Del Hospital Clínico San Carlos, 28040 Madrid (ES)
(72) Inventor: GONZÁLEZ GUEDE, Irene, Madrid (ES); FERNÁNDEZ GUTIÉRREZ, Benjamín, Madrid (ES)
(74) Representative: Elion IP, S.L.

(57) **Abstract**

The present disclosure relates to the field of tissue on a chip, in particular it relates to a system comprising an osteochondral tissue on a chip and to a method for culturing osteochondral tissue on a chip, using such a system.

## Description

### TECHNICAL FIELD

The present disclosure relates to the field of tissue on a chip, in particular it relates to a system comprising an osteochondral tissue on a chip and to a method for culturing osteochondral tissue on a chip, using such a system.

### BACKGROUND

Osteoarthritis is a disease characterized by progressive deterioration and loss of articular cartilage with concomitant structural and functional changes throughout the joint, including the synovial membrane, menisci, periarticular ligaments, and subchondral bone. The manifestations in patients suffering from the disease are pain, stiffness, reduced movement, swelling and joint crepitus. Osteoarthritis does not have a defined pharmacological treatment, with the most commonly used pharmacological treatment being NSAIDs (non-steroidal anti-inflammatory drugs) and, in cases of pharmacological failure, joint replacement surgery.

Globally, osteoarthritis is the leading cause of disability, with a dynamic prevalence increasing due to an aging population and increased risk factors. These risk factors include, for instance, advanced age, female sex, genetic predisposition, obesity and lifestyle, among others. Consequently, osteoarthritis entails a great socioeconomic impact, with high direct costs both in medication, medical visits, hospital treatments and joint replacement operations, as well as in indirect costs related to absenteeism from work and disability payments.

To address this problem, research efforts have focused on finding treatments or medication to reduce or reverse the effects of osteoarthritis. To evaluate the effects such treatments and medication there is a need for *in vitro* models of osteoarthritis. However, current *in vitro* models have many limitations in reflecting the real complexity of osteochondral tissues. Currently, three-dimensional cell cultures are being proposed to better mimic morphology and physiological functions of tissues, obtaining more reliable results. However, given the joint complexity, not all 3D cultures and co-cultures proposed for osteoarthritis reflect the pathophysiology of the disease and others require better validation of the models. Reference is made to, e.g., Muenzebrock et al. 2022 which have reviewed co-culture systems for osteoarthritis research (Muenzebrock KA, Kersten V, Alblas J, Garcia JP, Creemers LB, "The Added Value of the "Co" in Co-Culture Systems in Research on Osteoarthritis Pathology and Treatment Development', Front. Bioeng. Biotechnol.; March 2022; Vol. 10; Article 843056; [DOI: 10.3389/fbioe.2022.843056]).

On the other hand, with respect to *in vivo* studies there is no single standard animal model that accurately reflects the aspects of osteoarthritis in humans. Although the most commonly used models are small animals, ultimately, the results need to be confirmed in large animals (dog, sheep or horse) because of their higher similarity to human anatomy. These large animals pose disadvantages related to handling, cost, slower disease progression, and ethical considerations.

In view of the limitations of the available models for the study of osteoarthritis, it is necessary to find a more reliable, accessible and reproducible model in order to advance in the research of this disease.

### SUMMARY

In recent years, an advanced 3D culture technology known as Organ and Tissue on a Chip has been developed. These are microfluidic 3D cell culture devices that contain continuous infusion chambers, ensuring optimal nutrient delivery and preventing the accumulation of waste products. This novel technology has shown that it is capable of effectively maintaining cells and tissues, recreating their microenvironments and cellular interactions, and developing organs on a miniaturized scale, proposing them as a substitute for the animal model.

It has now been found that osteochondral tissue may be suitably cultivated on a chip, offering a model for studying osteochondral tissue and a research platform for osteoarthritis.

In particular, the present disclosure relates to an osteochondral tissue on a chip system (100) comprising:
a) an osteochondral tissue sample (110) previously extracted from a subject, the osteochondral tissue comprising a subchondral bone layer (111) together with a cartilage layer (112); and
b) a chip (120) comprising from bottom to top:
   i) a culture medium chamber (121), having an inlet and an outlet allowing a culture medium to flow through the culture medium chamber;
   ii) a membrane (122);
   iii) a tissue chamber (123); and
   iv) a cover (124);
   the membrane (122) placed between the culture medium chamber (121) and the tissue chamber (123) separating said chambers, and the cover (124) sealing the tissue chamber (123);
wherein the osteochondral tissue (110) is placed in the tissue chamber having the subchondral bone layer (111) placed directly on top of the membrane and the cover placed on top of the cartilage layer (112).

It has been found that such a tissue on a chip system and configuration is particularly suitable for the culture of an osteochondral tissue. Without being bound to any theory, such as system and configuration would be able to mimic physiological conditions in that the culture medium placed underneath the subchondral bone layer of the osteochondral tissue would not directly contact the cartilage layer, thereby the cartilage would get its nutrients through said subchondral layer, as it would be the case in vivo.

Accordingly, the present disclosure further relates to a method for culturing an osteochondral tissue on a chip comprising culturing an osteochondral tissue sample (110) using said system (100), comprising allowing a culture medium to flow through the culture medium chamber.

### BRIEF DESCRIPTION OF THE DRAWINGS

To complete the description and in order to provide for a better understanding of the disclosure, a set of drawings is provided. Said drawings form an integral part of the description and illustrate embodiments of the disclosure, which should not be interpreted as restricting the scope of the disclosure, but just as examples of how the disclosure can be carried out. The drawings comprise the following figures:

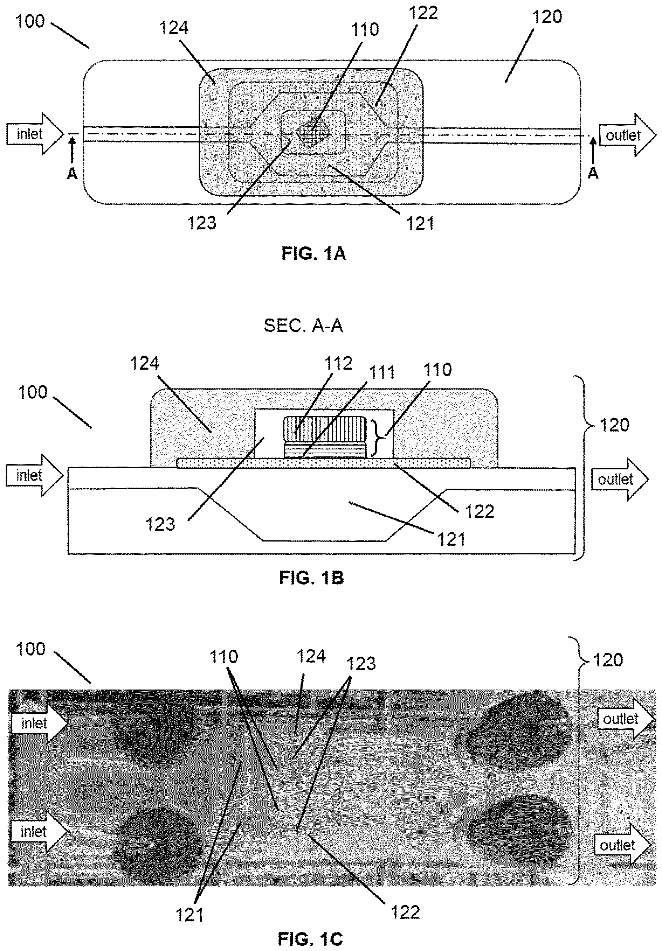

Figure 1 shows a schematic representation of an osteochondral tissue on a chip system (100) on its top view (Figure 1A) and on its longitudinal cross-section view (Figure 1B, section A-A of Figure 1A) and also shows a picture of an osteochondral tissue on a chip system (100) having two sets of chambers and two osteochondral tissue samples (Figure 1C).

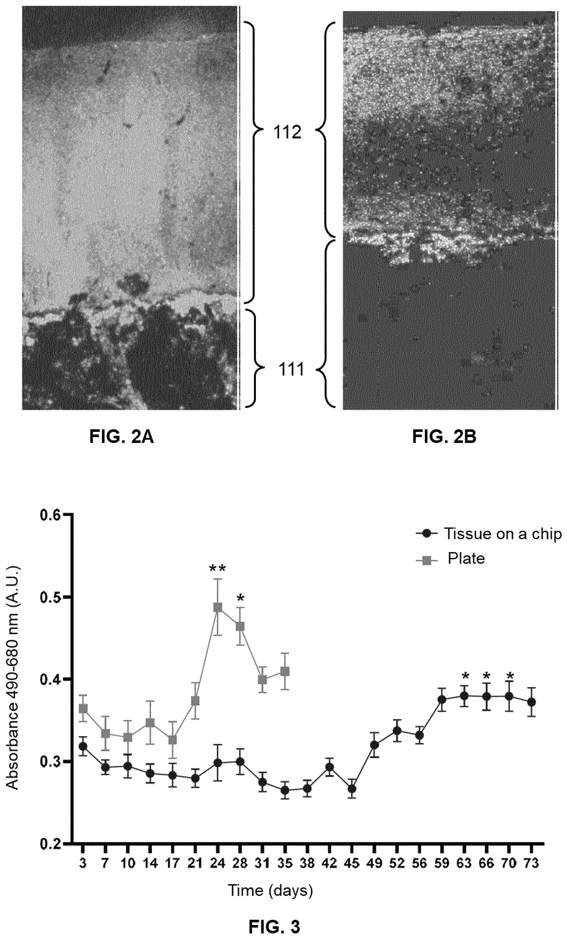

Figure 2 shows two samples of osteochondral tissue cultured on a chip as described herein treated to show the distribution of glucose (Figure 2A) and the distribution of oxygen (Figure 2B) as the lighter areas in the tissue, where the difference in distribution can be clearly observed for the two regions of the tissue relating to the subchondral bone layer (111) and the cartilage layer (112).
Figure 3 shows a graph of the absorbance at 490-680 nm over time, as a measure of the presence of lactate dehydrogenase (LDH) which provides an indication of cellular cytotoxicity in the growth medium obtained after culturing two samples of osteochondral tissue: one cultured on a chip as described herein and the other one cultured on a plate.

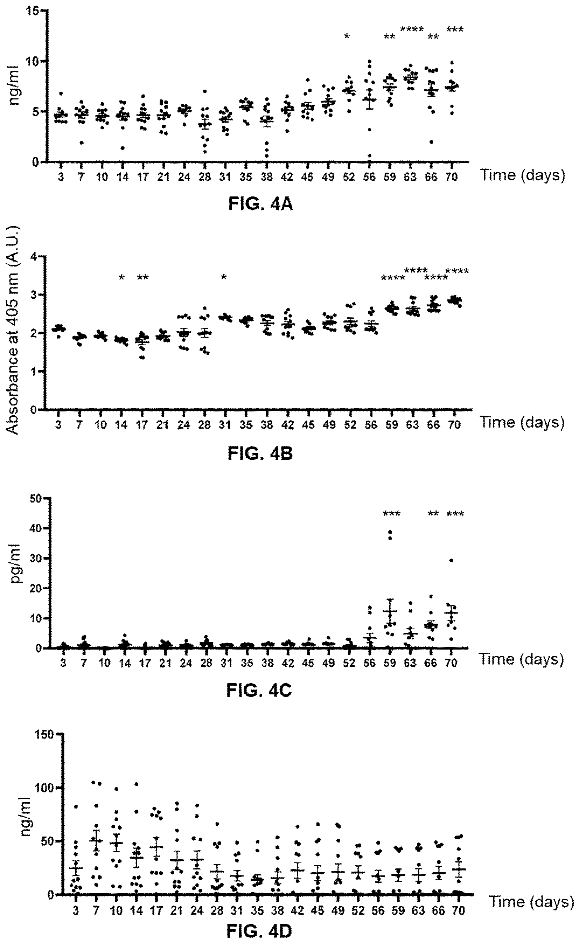

Figure 4 shows the stability of the osteochondral tissue over 70 days of culture on a chip as described herein, by measure of: the concentration of glycosaminoglycans (GAGs) (ng/ml) in the culture medium as a measure of degradation enzyme activity in cartilage (Figure 4A); alkaline phosphatase (ALP) activity as a measure of the activity of osteoblast by measuring absorbance at 405 nm of the culture medium in the presence of p-Nitrophenyl Phosphate (PNPP) phosphatase substrate (Figure 4B); the concentration of type II collagen peptide (Coll2-1) (pg/ml) in the culture medium, as a measure of cartilage degradation (Figure 4C); and the concentration of procollagen II N-terminal propeptide (PIINP) (pg/ml) in the culture medium, as a measure of the synthesis of new collagen.

### DETAILED DESCRIPTION

As specified above the present disclosure relates to an osteochondral tissue on a chip system (100) comprising:
a) an osteochondral tissue sample (110) previously extracted from a subject, the osteochondral tissue sample comprising a subchondral bone layer (111) together with a cartilage layer (112); and
b) a chip (120) comprising from bottom to top:
   i) a culture medium chamber (121), having an inlet and an outlet allowing a culture medium to flow through the culture medium chamber;
   ii) a membrane (122);
   iii) a tissue chamber (123); and
   iv) a cover (124);

the membrane (122) being placed between the culture medium chamber (121) and the tissue chamber (123) and separating said chambers,
the cover (124) sealing the tissue chamber (123) and
wherein the osteochondral tissue (110) is placed in the tissue chamber (123) having the subchondral bone layer (111) placed directly on top of the membrane (122) and the cover (124) placed on top of the cartilage layer (112).

Particular embodiments of such a system are illustrated in Figure 1. For instance, Figure 1A shows a top view a schematic representation of an osteochondral tissue on a chip system (100), comprising an osteochondral tissue sample (110) and a chip (120), wherein the osteochondral tissue sample (110) is placed in the tissue chamber (123), with arrows indicating the inlet and outlet of the culture medium chamber, where the culture medium may enter and exit. Figure 1B shows a longitudinal cross-section view, in particular section A-A of Figure 1A, where in addition to the inlet and outlet from the culture medium chamber, the osteochondral tissue (110) can be seen placed in the tissue chamber having the subchondral bone layer (111) placed directly on top of the membrane (122) and the cover (124) placed on top of the cartilage layer (112). Figure 1C shows a picture of a particular osteochondral tissue on a chip system (100) having two sets of chambers and two osteochondral tissue samples, the picture also shows tubes that can be used to provide the medium entering and exiting the culture medium chamber as marked by the inlet and outlet arrows.

Osteochondral tissue as used herein has the meaning currently used in the art and refers to tissue found at joint surfaces (e.g., synovial joint surfaces) and composed of both articular cartilage and subchondral bone.

An osteochondral tissue sample (110) refers to a sample of osteochondral tissue previously extracted from a subject. In a system and methods described herein, the osteochondral tissue sample (110) comprises both a subchondral bone layer (111) and a cartilage layer (112), in particular comprises these two layers together. More in particular, these two layers form a continuous tissue, as they are found in their physiological environment, and have not been separated from each other.

In several particular embodiments the subchondral bone layer (111) may have a thickness from 0,5 to 6 mm in particular from 1 to 3 mm, and/or the cartilage layer (112) may have a thickness from 1 to 6 mm, in particular from 1 to 4 mm.

The sample of osteochondral tissue may be of any suitable dimensions. For instance, may be sufficiently large to encompass both the subchondral bone layer (111) and a cartilage layer (112) and its subsequently culture and sufficiently small to enable handling and positioning on a chip.

As a mode of example, the osteochondral tissue sample (110) may have the following dimensions:
- from 0.7 to 10 mm long, in particular from 1 to 8 mm and more in particular from 2 to 5 mm long
- from 0.7 to 10 mm wide, in particular from 1 to 8 mm and more in particular from 2 to 5 mm wide
- from 0.7 to 20 mm high, more particular from 1 to 15 mm and more in particular from 2 to 5 mm high.

An osteochondral tissue sample (110) previously extracted from a subject may have been previously extracted by any means known in the art.

The subject from which the osteochondral tissue sample (110) has been extracted may be a human or an animal subject, in particular a human subject, more in particular a healthy human subject or a human subject suffering from osteoarthritis. The human or animal subject may be a living or may be dead.

In several embodiments, the osteochondral tissue sample (110) may have been extracted from a joint of the subject, in particular from a synovial joint, and more in particular from a synovial joint selected from: compound joints, such as a knee joint; plane joints, such as a wrist carpal joint; hinge joints, such as an elbow joint; pivot joints such as an atlanto-axial joint, a proximal radioulnar joint, and a distal radioulnar joint; condyloid joints, such as a wrist radiocarpal joint; and ball and socket joints, such as a shoulder joint and a hip joint.

As a mode of example, a sample of osteochondral tissue may have been previously extracted from osteoarthritis human patients undergoing joint replacement, e.g., knee joint replacement. The sample may be obtained during the performance of this surgery. In particular, osteochondral tissue samples may be extracted and cut into pieces in the operating room with surgical material. The extracted samples may then be stored at 4 °C, preferably for less than 24 hours, in multi-well plates with culture medium, prior to being used on a chip to provide a system (100) as described herein. The tissue samples may be modeled for culture by cutting them to an appropriate size, for instance (3 x 3 x 4) mm (length x width x height).

An osteochondral tissue on a chip system (100) comprises a chip (120) with a specific configuration. In particular, a chip may comprise from bottom to top:
i) a culture medium chamber (121);
ii) a membrane (122);
iii) a tissue chamber (123); and
iv) a cover (124).

In a chip (120) of a system (100) as described herein, the culture medium chamber (121) has an inlet and an outlet allowing a culture medium to flow through the culture medium chamber. The inlet and/or outlet may be connected to a tube to feed the culture medium to the culture medium chamber, and/or to collect culture medium from the culture medium chamber. The culture medium chamber (121) may be connected to a pump, e.g., through said inlet or outlet. The pump may facilitate regulating the flow of culture medium, as detail further below. As a mode of example a perfusion pump, such as a commercial New Era Pump System, may be used.

The culture medium chamber (121) may be of dimensions suitable for retaining a sufficient amount of culture medium to allow the exchange of nutrients and waste products (such as metabolites) with the osteochondral tissue placed in the tissue chamber (123) above. In several embodiments the culture medium chamber may be bigger than the tissue chamber. Furthermore, the culture medium chamber may be connected to the channels extending from the culture medium chamber through the inlet and outlet, which channels may be connected to feeding and collecting tubes as described above. Said channels and said tubes may themselves be also referred to as inlet and outlet respectively to refer to the channels and tubes that provide the incoming medium and those that provide the outgoing medium.

As a mode of example, the culture medium chamber (121) may have the following dimensions:
- from 1 to 50 mm long, in particular from 2 to 35 mm and more in particular from 3 to 25 mm long
- from 1 to 50 mm wide, in particular from 2 to 35 mm and more in particular from 3 to 25 mm wide
- from 1 to 25 mm high, more particular from 2 to 20 mm and more in particular from 3 to 10 mm high.

In a chip (120) of a system (100) as described herein, the membrane (122) is placed between the culture medium chamber (121) and the tissue chamber (123) separating said chambers. Thereby, the culture medium chamber and the tissue chamber communicate only through said membrane and when a culture medium flows through the culture medium chamber, the membrane may physically separate the osteochondral tissue placed in the tissue chamber (123) and the culture medium flowing through the culture medium chamber (121).

The membrane (122) may have dimensions to cover, at least, the part of the culture medium chamber that communicates with the tissue chamber. On the other hand, membrane will cover all the surface of the tissue chamber that is in contact with the medium chamber, which will generally be all the surface of the tissue chamber. Therefore, the dimensions of the membrane may be of at least the size of the surface of the tissue chamber.

As a mode of example, the membrane (122) may have the following dimensions:
- from 1 to 70 mm long, in particular from 4 to 60 mm and more in particular from 5 to 50 mm long
- from 1 to 70 mm wide, in particular from 2 to 60 mm and more in particular from 3 to 50 mm wide
- from 0.01 to 1 mm high, more particular from 0.05 to 0.75 mm and more in particular from 0.1 to 0.5 mm high.

The membrane may allow the exchange of nutrients (such as glucose and oxygen) and waste products (such as metabolites) between the osteochondral tissue and the culture medium. For instance, the incoming culture medium rich in nutrients (such as glucose and oxygen) may provide nutrients to the osteochondral tissue placed in the tissue chamber when flowing through the culture medium chamber placed underneath the tissue chamber. At the same time, the culture medium may collect waste products from the osteochondral tissue. Consequently, the outgoing culture medium may be enriched in said waste products and impoverished in nutrients.

In a chip (120) of a system (100) as described herein, the tissue chamber (123) is placed on top of the culture medium chamber (122) separated thereof by the membrane. The tissue chamber may be of dimensions suitable for hosting a sample of osteochondral tissue and to allow the exchange of nutrients and metabolites with the culture medium flowing through the culture medium chamber (121) situated below. In several embodiments tissue chamber may be smaller than the culture medium chamber.

As a mode of example, the tissue chamber (123) may have the following dimensions:
- from 1 to 40 mm long, in particular from 2 to 30 mm and more in particular from 3 to 20 mm long
- from 1 to 40 mm wide, in particular from 2 to 30 mm and more in particular from 3 to 20 mm wide
- from 1 to 40 mm high, more particular from 2 to 30 mm and more in particular from 3 to 20 mm high.

In a chip (120) of a system (100) as described herein, the cover (124) seals the tissue chamber (123). That the cover seals the tissue chamber is understood herein as not allowing any exchange, e.g., of oxygen, to occur between the tissue chamber and its surrounding environment through the cover.

The cover (124) may be an adhesive cover. An adhesive cover may be preferred as it may facilitate the seal of the tissue chamber. For instance, the cover may be of a polymeric material comprising an adhesive on the side on which it contacts the tissue chamber.

The cover (124) may have dimensions to cover, at least, the part of the tissue chamber exposed to the surrounding environment. As a mode of example, the cover (124) may have the following dimensions:
- from 2 to 70 mm long, in particular from 4 to 60 mm and more in particular from 6 to 50 mm long
- from 2 to 70 mm wide, in particular from 4 to 60 mm and more in particular from 6 to 40 mm wide
- from 0.01 to 5 mm high, more particular from 0.5 to 4 mm and more in particular from 0.1 to 3 mm high.

Different parts of the chip (120) may be composed of different materials. Materials of the chip may preferably be medical grade materials. In several embodiments the chip (120) may comprise a material with low permeability to oxygen. In particular, a material with low permeability to oxygen may be a material impermeable to oxygen. A material with low permeability to oxygen is understood as a material allows small amounts of oxygen to go through the material and a material impermeable to oxygen is a material that does not allow oxygen to go through the material. For instance, a material with low permeability to oxygen may have an oxygen permeability from 0.5 to 15 cc/m² every 24hr, as measured by the ASTM standard D3985, in particular from 0.5 to 5 cc/m² every 24hr.

A material with low permeability to oxygen may be polymeric material with low permeability to oxygen, and more in particular may be a polymeric material selected from a cyclic olefin polymer (COP), cyclic olefin copolymer (COC) and polyethylene terephthalate (PET).

In several particular embodiments, the culture medium chamber (121); the tissue chamber (123); and the cover (124) may comprise or may be constituted of such materials with low permeability to oxygen. Other parts of the chip, for instance tubes connected to the inlet and outlet of the culture medium chamber may also be of such materials. Such materials may be useful to ensure that the composition of the culture medium has a controlled amount of oxygen and that the osteochondral tissue placed in the tissue chamber does not absorb oxygen from its surrounding environment, thereby ensuring that, e.g., all the oxygen arriving to the tissue arrives via de culture medium.

In contrast, the membrane (122) may be of an oxygen permeable material, allowing for oxygen and preferably also other nutrients (such as oxygen and glucose) or waste products (such as metabolites) to go through the membrane.

In several embodiments, the membrane (122) may be of a porous material, in particular, a porous material having a pore size from 0.1 to 20 microns, in particular from 0.2 to 15 microns, more in particular from 0.4 to 10 microns. The pore size may be known from the supplier of the porous material or may be determined by methods known in the art. For instance, the pore size may refer to the average pore size as determined by, e.g., mercury intrusion porosimetry or scanning electron microscopy (SEM). Suitable porous materials may be selected from, e.g., polycarbonate, polyethylene terephthalate (PET), polytetrafluoroethylene (PTFE), or polyethersulfone (PES).

In several embodiments in a system (100) as described herein the culture medium chamber (121) may comprise a culture medium. A culture medium is understood as a liquid composition, in particular an aqueous composition, comprising nutrients for the culture of cells, in particular a tissue such as an osteochondral tissue.

The culture medium may generally comprise water, glucose and oxygen. As a mode of example, a culture medium may comprise
- from 0.05 to 5 g/L of D-glucose, in particular from 0.5 to 2 g/L and more in particular about 1 g/L of D-glucose, and/or
- from 3 to 14 mg/L of oxygen, in particular from 6 to 11 mg/L of oxygen.

A culture medium may be supplemented with other components. Accordingly, the culture medium may optionally comprise additional components selected from, e.g., amino acids (e.g., glutamine or glutamine precursors), vitamins, inorganic salts, pyruvate (e.g., sodium pyruvate), antibiotics (e.g., penicillin-streptomycin) and fetal bovine serum.

A culture medium suitable for a system (100) as described herein may be a culture medium for mammal cells. Culture medium for mammal cells may be commercially available and may be prepared according to the supplier instructions. As a mode of example, a commercial Dulbeccos Modified Eagle Medium (DMEM) may be used, having, e.g., about 1 g/L of glucose, and supplemented with a glutamine precursor (e.g., GlutaMAX), sodium pyruvate, penicillin-streptomycin (e.g. about 1%) and fetal bovine serum (e.g., up to 15 %, in particular about 10 %).

The present disclosure further relates to a method for culturing an osteochondral tissue on a chip comprising culturing an osteochondral tissue sample (110) using a system (100) as described herein.

The method comprises allowing a culture medium, such as a culture medium described in detail above, to flow through the culture medium chamber of the system (100). As indicated above the flow of culture medium through the culture medium chamber (121) allows for the exchange of nutrients and waste products between the culture medium and the osteochondral tissue placed in the tissue chamber (123) through the membrane (122).

A method as described herein allows for the osteochondral tissue to be cultured under conditions that mimic physiological conditions. In particular, the disposition of the tissue in the chip of the system and in particular relative to the culture medium and its surrounding environment, allow for the different layers of the tissue, in particular the subchondral bone layer (111) and the cartilage layer (112), to reach conditions similar to those reached under physiological conditions as it will be explained in more detail below.

In a method as described herein the system (100) may be preferably maintained at a temperature from 35 to 39 °C, in particular from 36 to 38 °C. Such temperatures may be preferred as they are close to the physiological temperature of the human body.

The culture medium may flow through the culture medium chamber at a flow rate from 0.1 to 10 µl/min, in particular from 0.5 to 5 µl/min, more in particular from 1 to 3 µl/min. Such flow rates allow have been found to contribute to the exchange of nutrients and waste products between the culture medium and the osteochondral tissue. Such flow rates may be achieved by connecting the system to a pump, e.g., a perfusion pump, as described above.

Using a method as described here in culturing may be performed from, e.g., 5 to 90 days, in particular from 10 to 70 days, more in particular from 15 to 60 days yet more in particular from 20 to 50 days.

It has been found that a method as described herein may allow to culture osteochondral tissue for a longer period of time than other methods, whilst maintaining the stability of the tissue and the viability of the cells, as also described and shown in more detail below.

In several embodiments in a method as described herein the culture medium may be supplied as an incoming culture medium to the culture medium chamber (i) through the inlet of the culture medium chamber and the culture medium is collected as an outgoing culture medium through the outlet of the culture medium chamber. Thereby, the culture medium may provide nutrients from the incoming culture medium to the osteochondral tissue sample (110) through the membrane (122) and the culture medium may collect waste products from the osteochondral tissue sample (110) through the membrane (122) to provide the outgoing culture medium. Nutrients may include, e.g., oxygen and glucose among other components such as amino acids, vitamins, inorganic salts and pyruvate as discussed above. Waste products may include, e.g., metabolites such as lactate, and other waste products such as glycosaminoglycans (GAGs), type II collagen peptide (Coll2-1), procollagen II N-terminal propeptide (PIINP), matrix metalloproteinases (MMPs), and prostaglandins.

The composition of the incoming culture medium may typically be different from the composition of the outgoing culture medium. Generally, the incoming culture medium may be rich in nutrients (as described above) and the outgoing culture medium may be enriched in waste products and impoverished in nutrients.

In particular, in a method as described herein the outgoing culture medium may have a concentration of glycosaminoglycans (GAGs) of at least 0.25 ng/ml. The amount of glycosaminoglycans provides a measure of degradation of enzymes in the cartilage, a low concentration of GAGs in the outgoing culture medium denotes low enzyme degradation. It has been found that the concentration of GAGs in the outgoing culture medium may be maintained relatively low with increasing culture time, and it may typically increase over time. For instance, the concentration of GAGs may be of at most 10 ng/ml, preferably at most 8 ng/ml, e.g., after 70 days of culture; or may be of at most 8 ng/ml, preferably at most 7 ng/ml, e.g., after 50 days of culture or at most 7 ng/ml, preferably 5 ng/ml after 15 days of culture. The GAGs concentration may be determined by means known in the art, for instance, using a commercially available kit such as Human GAGs (Glycosaminoglycan) ELISA kit, e.g., available from MyBiosource (Catalog No: E-EL-H2598).

In a method as described herein the outgoing culture medium may have a variable alkaline phosphatase (ALP) activity as an indication of an adequate activity of osteoblast. For instance, an ALP activity that is signifcantly reduced with time may be indicative of a reduced osteoblast activity and a markedly increased ALP activity may be indicative of an abnormal osteoblast activity, e.g., due to an abnormal bone remodeling. The ALP activity may be assessed by measuring absorbance at 405 nm of the culture medium in the presence of a phosphatase substrate, which may be commercially available, such as p-Nitrophenyl Phosphate (PNPP) from ThermoFisher (Catalog Number Ref.: 37620). In particular, an outgoing culture medium may have an absorvance from 1 to 3 A.U..

In a method as described here in the outgoing culture medium may have a concentration of type II collagen peptide (Coll2-1) from 0.1 to 40 pg/ml. The concentration of Coll2-1 may be measured by means known in the art, for instance a commercially available kit, such as Human Coll2-1 Elisa Kit from Cusabio (Catalog No: CSB-EQ027311HU), may be used. The amount of Coll2-1 provides a measure of cartilage degradation, which may typically increase with the culture time. Therefore, a low concentration of Coll2-1 in the outgoing culture medium may denote low cartilage degradation and stability and viability of the osteochondral tissue. It has been found that the concentration of Coll2-1 in the outgoing culture medium may be maintained relatively low with increasing culture time. For instance, the concentration of Coll2-1 in the outgoing culture medium may be of at most 20 pg/ml, preferably of at most 15 pg/ml after 70 days of culture; or may be of at most 10 pg/ml, preferably at most 8 pg/ml, e.g., after 50 days of culture; or may be of at most 5 pg/ml, preferably at most 3 pg, e.g., after 15 days of culture.

In a method as described herein the outgoing culture medium may have and the concentration of procollagen II N-terminal propeptide (PIINP) from 0.1 to 125 pg/ml in the outgoing culture medium. The concentration of PIINP may be measured by means known in the art, for instance a commercially available kit, such as Human Procollagen II N-Terminal Propeptide (PIINP) ELISA Kit from Bioss Antibodies (Catalog No: BSKH61221) may be used. The concentration of PIINP, which may typically decrease with the culture time, may be used as a measure of the synthesis of new collagen and therefore an indication of the viability of the cells and the health of the osteochondral tissue. It has been found that the concentration of PIINP in the outgoing culture medium may be maintained relatively high with increasing culture time. For instance, the concentration of PIINP may be of at least 5 pg/ml, preferably 10 pg/ml, after, e.g., 70 days of culture; or may be of at least 12 pg/ml, preferably at least 14 pg/ml, after, e.g., 50 days of culture; or may be of at least 20 pg/ml, preferably at least 25 pg/ml, after, e.g., 15 days of culture.

Given the ability to culture osteochondral tissue for long periods of time whilst maintaining its stability and viability, methods for culturing osteochondral tissue as described herein may be useful for evaluating pharmaceutical agent and/or the diagnostic agent on an osteochondral tissue sample (110).

Accordingly, in several embodiments a method as described herein may comprise, e.g., administering a pharmaceutical agent, a diagnostic agent and/or a metabolic agent to the osteochondral tissue sample (110).

A pharmaceutical agent is understood herein as commonly understood in the art, e.g., as a substance that provides a therapeutic effect when administered to a subject. A pharmaceutical agent may include, e.g., a small molecule or a biological product, such as a protein or peptide. As a mode of example, a pharmaceutical agent may be particularly selected from inhibitors of Wnt (Wingless and Int-1) signaling, modulators of the β-catenin pathway, anti-inflammatory compounds, cartilage regeneration promoters, or osteoclast inhibitors, in particular, small molecule inhibitors of GSK3, monoclonal antibodies against sclerostin, or recombinant proteins that enhance chondrocyte proliferation and matrix production.

A diagnostic agent is understood herein as commonly understood in the art, e.g., as a substance that may be used to examine the body in order to detect or to rule out impairment of its normal functions. As a mode of example, a diagnostic agent may be selected from imaging agents, biomarkers, contrast agents, or molecular probes, in particular MRI contrast agents, specific antibodies targeting biomarkers of cartilage degradation, or fluorescent probes for detecting inflammatory markers.

A metabolic agent is understood herein as commonly understood in the art, e.g., as a substance capable of producing an effect on the sum of the chemical and physical changes occurring in tissue. As a mode of example, a metabolic agent may be selected from enzyme inhibitors, or metabolic modulators, in particular insulin-like growth factor (IGF), interleukin-1 receptor antagonists, or matrix metalloproteinase inhibitors.

Administering such agents in a method as described herein may preferably comprise supplying a culture medium as an incoming culture medium, e.g., as described above, wherein the incoming culture medium comprises the pharmaceutical agent the diagnostic agent and/or the metabolic agent. For instance, said agent may be dissolved in the culture medium or may be incorporated to the culture medium in a vehicle for the delivery and/or preservation of drugs, e.g., encapsulated in a particle.

In several embodiments a method as described herein may further comprise monitoring the effect of the pharmaceutical agent, the diagnostic agent and/or metabolic agent on the osteochondral tissue sample (110). For instance, the effect of a pharmaceutical agent, diagnostic agent and/or metabolic agent on an osteochondral tissue of a human patient suffering from osteoarthritis may be monitored by evaluating some property of the tissue before and after the administration of said agent.

In several particular embodiments, monitoring may comprise, e.g., directly evaluating the osteochondral tissue sample (110), preferably using optical means. In particular, monitoring may include the use of confocal microscopy to assess the structural integrity and cellular health of the tissue, e.g., visualization of cellular morphology, extracellular matrix organization, and any pathological changes associated.

Additionally, or alternatively, in several particular embodiments, monitoring may comprise, e.g., indirectly evaluating the osteochondral tissue sample (110), preferably analyzing the composition of the culture medium, more preferably analyzing the composition of the outgoing culture medium provided as described above. In particular, indirect evaluation may include the use of enzyme-linked immunosorbent assay (ELISA) to detect and quantify biomarkers such as collagen degradation products, inflammatory cytokines, and matrix metalloproteinases in the culture medium.

In this text, the term "includes", "comprises" and derivations thereof (such as "including", "comprising", etc.) should not be understood in an excluding sense, that is, these terms should not be interpreted as excluding the possibility that what is described and defined may include further elements, steps, etc.

On the other hand, the disclosure is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

The present disclosure is further illustrated by the following examples without being limited thereto or thereby.

### EXAMPLES

### Example 1: Osteocondral tissue on a chip system

A commercially available chip (a Be-Transflow chip from BeOnChip) was used made of COP and COC, which are medical grade plastics with a very low oxygen permeability. The chip had two culture medium chambers and two tissue chambers. An 8-micron pore-sized membrane was placed between the culture medium chamber at the bottom of the chip, through which flowed through the culture medium was made to flow as explained below, and the tissue chamber. An osteochondral tissue sample was be placed on the chip with the subchondral bone layer on the membrane. After a sample of osteochondral tissue was inserted, an adhesive cover was placed to provide a completely closed tissue chamber, with access to the culture medium of the culture medium chamber through the membrane. Each culture medium chamber was connected to an inlet and an outlet PET tube, which also has low gas permeability. Each PET tube was provided with a rubber stopper, to completely close the system. A syringe with a 20G needle was inserted into the rubber stopper of the inlet of the culture medium chamber. This syringe was placed in a perfusion pump (New Era Pump System), where the flow rate of 2 µl/min was selected. The medium flowed from the syringe through the culture medium chamber at the selected perfusion rate. The perfused medium was collected through the outlet rubber in a tube for further analysis.

A schematic representation picture of the osteochondral tissue on a chip system used can be seen in Figure 1 including a schematic representation of an osteochondral tissue on a chip system (100) on its top view (Figure 1A) and on its longitudinal cross-section view (Figure 1B, section A-A of Figure 1A). Figure 1 also shows a picture of an osteochondral tissue on a chip system (100) having two sets of chambers and two osteochondral tissue samples (Figure 1C). In figures 1A-1C both the osteochondral tissue sample (110) and the chip (120) are depicted. The osteochondral tissue sample (110) comprising a subchondral bone layer (111) together with a cartilage layer (112). The chip (120) comprising from bottom to top:
i) a culture medium chamber (121), having an inlet and an outlet allowing a culture medium to flow through the culture medium chamber;
ii) a membrane (122);
iii) a tissue chamber (123); and
iv) a cover (124);

the membrane (122) placed between the culture medium chamber (121) and the tissue chamber (123) separating said chambers, and the cover (124) sealing the tissue chamber (123);
wherein the osteochondral tissue (110) is placed in the tissue chamber having the subchondral bone layer (111) placed directly on top of the membrane and the cover placed on top of the cartilage layer (112).

Using the prepared osteochondral tissue on a chip system as illustrated in Figure 1 nutrients were constantly provided to the osteochondral tissue sample through the inlet with the incoming culture medium and any molecules of interest. Waste products, e.g. metabolites, generated in the culture itself were collected through the outlet in the outgoing culture medium.

In addition, the use of a gas-impermeable system ensured that the oxygen supplied to the osteochondral tissue sample came exclusively from the oxygen dissolved in the culture medium and was controlled by the selected flow rate. With the subchondral bone layer of osteochondral tissue sample the being placed on the membrane, it was said subchondral bone layer which distributed nutrients and oxygen to the cartilage layer, mimicking the physiological environment, where the cartilage, being an avascular tissue, also gets its nutrients and oxygen through its subchondral bone layer.

### Samples

Human samples of osteochondral tissue comprising both the subchondral bone layer and the cartilage layer were previously extracted from patients diagnosed with osteoarthritis based on the ACR (American College of Rheumatology) criteria of 1992, who had undergone joint replacement surgery and were obtained during this surgery. Previously, the donor patients had given their informed consent through a form duly signed and the extraction and use of the samples was performed respecting the ethical principles of medical research on human subjects, including research on identifiable human material and information, in accordance with the Declaration of Helsinki.

Osteochondral tissues were cut into pieces in the operating room with surgical material and were stored at 4°C for less than 24 hours in multiwell plates with culture medium. Subsequently, the tissues were modeled for culture to a size (3 x 3x4) mm, (length x width x height) using an acrylic cutting block with 1mm sections and a carbon steel knife as a guide.

### Example 2: Fluorescence in living tissue cells

To evaluate the distribution of glucose in tissues cultured with the system of example 1, osteochondral tissues from two patients were used. The tissues were treated with 2-NBDG (2-(N-(7-nitrobenz-2-oxa-1,3-diazole-4-yl)amino)-2-deoxyglucose) (ref.: N13195, Thermofisher), which is a fluorescent glucose analogue that is used to monitor glucose consumption in living cells, as an indicator of cell viability. Another indicator of cell viability used is DAPI (4', 6-diamidino-2-phenylindole) (Ref: 62248, Thermofisher) which is a fluorescent stain that binds strongly to adenine-thymine-rich regions in DNA used extensively in fluorescence microscopy. As DAPI can pass through an intact cell membrane, it can be used to stain both live and fixed cells, though it passes through the membrane less efficiently in live cells and therefore provides a marker for membrane viability. To monitor the distribution of oxygen in the cultured tissues Image-iT Green Hypoxia Reagents (Thermofisher) was used, which is a reagent which is fluorogenic when the level of oxygen goes below 5%, being an indicator of hypoxia when it fluoresces (low oxygen concentration in a tissue) and of normoxia (normal level of oxygen in a tissue) when it does not fluoresce.

Figure 2 shows said two samples of osteochondral tissue cultured on the chip as described herein treated to show the distribution of glucose (Figure 2A) and the distribution of oxygen (Figure 2B) as the lighter areas in the tissue, where the difference in distribution can be clearly observed for the two regions of the tissue relating to the subchondral bone layer (111) and the cartilage layer (112).

From Figure 2A it can be clearly observed that glucose arrived to the cartilage layer placed above the subchondral bone layer, and that the distribution of said glucose was homogeneous throughout the cartilage layer whereas was not homogeneous in the subchondral bone layer. This is the same behavior that one would expect from the respective layers in a physiological environment.

From Figure 2B it can be clearly observed that that the cartilage layer (112) is in hypoxia (for being fluorescent green when treated with Image-iT and being the light fraction of the tissue in Figure 2B) and the subchondral bone layer (111) is in normoxia (for not being fluorescent when treated with Image-iT and being the dark fraction of the tissue in Figure 2B). This is the same behavior that one would expect from the respective layers in a physiological environment. On the other hand, the blue fluorescence by DAPI was only mildly observed in the treated osteochondral tissue (only perceived in Figure 2b as small spots with a dark outline in the subchondral bone layer (111) and the cartilage layer (112)) which also was an indication of the viability of the cells.

### Example 3: Comparison of osteochondral tissue culture on a chip and on a plate

Osteochondral tissue of the femur and tibia of the injured and non-injured parts of three patients (n= 24 osteochondral tissue samples). Twelve (12) samples were cultured in the osteochondral tissue culture on a chip system as described here in and as particularly described in example 1. Twelve (12) samples were cultured in parallel on plate according to established protocols. Reference is made to, e.g., Haltmayer *et al.* 2019 (Haltmayer E, Ribitsch I, Gabner S, Rosser J, Gueltekin S, Peham J, Giese U, Dolezal M, Egerbacher M, Jenner F, "Co-culture of osteochondral explants and synovial membrane as in vitro model for osteoarthritis."; PLoS One;. 2019 Apr 2; 14(4): e0214709. [DOI: 10.1371/journal.pone.0214709]; PMID: 30939166; PMCID: PMC6445514). Briefly, the osteochondral tissue samples were placed in multiwell plates of 12 wells. They were cultured in an incubator at 37°C and 5% CO₂, with culture medium and changed every 3 to 4 days.

Several properties of the tissue cultured on a chip were compared to those of the tissue cultured on a plate as detailed in the following.

### Lactate Deshidrogenasa (LDH)

The measurement of LDH in the culture medium used for the culture of the tissue provides an indication of cellular cytotoxicity. The LDH assessment was performed with the CyQUANT LDH Cytotoxicity Assay (ThermoFisher) kit, following the manufacturer's instructions.

The perfused medium of the outgoing culture medium obtained from chip was compared with the plate culture supernatant medium. The results are displayed in Figure 3, which shows a graph of the absorbance at 490-680 nm, as a measure of the presence of lactate dehydrogenase (LDH) which provides an indication of cellular cytotoxicity in the growth medium obtained after culturing. As it can be observed from Figure 3, an increase in LDH was observed at day 24 for the medium obtained from the plate culture and at day 63 for the medium obtained from the chip culture. Furthermore, the LDH level of the medium obtained from the plate culture was consistently higher than that obtained from the chip culture. This demonstrates a that degradation of the tissue is not only significantly postponed, but also consistently lower, when using a chip as described herein.

### Stability assessment of the tissue in an osteochondral tissue on a chip system

The stability of the osteochondral tissue cultured on a chip was also evaluated from the outgoing culture medium perfused on the chip by quantifying different molecules during 70 days of culture. The results are displayed in Figure 4.

The concentration of glycosaminoglycans (GAGs) (ng/ml) in the outgoing culture medium was evaluated as a measure of degradation enzyme activity in cartilage (Figure 4A). The Human GAGs were measured using ELISA kit commercially available from MyBiosource (catalog No: E-EL-H2598) following the manufacturer's instructions.

The alkaline phosphatase (ALP) activity in the outgoing culture medium was evaluated as a measure of the activity of osteoblast (Figure 4B). The ALP activity was evaluated by measuring absorbance at 405 nm of the culture medium in the presence of p-Nitrophenyl Phosphate (PNPP) phosphatase substrate commercially available from ThermoFisher (catalog Number Ref.: 37620), following the manufacturer's instructions.

The concentration of type II collagen peptide (Coll2-1) (pg/ml) in the outgoing culture medium was evaluated as a measure of as a measure of cartilage degradation (Figure 4C). The concentration of Human Coll2-1 was measured using an Elisa Kit commercially available from Cusabio (catalog No: CSBEQ027311HU), following the manufacturer's instructions.

The concentration of procollagen II N-terminal propeptide (PIINP) (pg/ml) in the outgoing culture medium was evaluated as a measure of the synthesis of new collagen (Figure 4D). The concentration of Human PIINP was measured using an Elisa Kit commercially available from Bioss Antibodies (catalog No: BSKH61221), following the manufacturer's instructions.

As it can be observed from Figure 4, compared to the beginning of culture:
- GAG concentration increased after 52 days (Figure 4A);
- ALP activity varied on days 14, 17, and 31, but increased markedly from day 59 onwards (Figure 4B);
- Coll2-1 concentration increased at 59 days (Figure 4C); and
- PIINP concentration remained stable throughout the 70 days of the culture (Figure 4D).

These examples demonstrate the validity of the osteochondral tissue on a chip system described herein as a model to reproduce the physiological microenvironment of the joints. For the first time, an environment of normoxia and hypoxia has been achieved in an osteochondral tissue in an easy-to-use device and without the need to apply nitrogen to limit oxygen in the cartilage.

Survival and tissue parameters were observed to be stable over approximately at least two months of culture (e.g., about 60-70 days). In addition, when the entire tissue was cultured, the existing cartilage-bone communication is not lost.

In contrast to other 3D and *in vivo* models, wherein the disease on the model tissue needs to be chemically, genetically or surgically induced and does not reflect all the characteristics of osteoarthritis, in the osteochondral tissue on a chip system described, the osteochondral tissue samples come from patients with osteoarthritis, being able to evaluate new drugs more effectively and being a valid proposal to replace animal models, although it could also be used to evaluate healthy tissue.

## Claims

1. An osteochondral tissue on a chip system (100) comprising:
a) an osteochondral tissue sample (110) previously extracted from a subject, the osteochondral tissue sample comprising a subchondral bone layer (111) together with a cartilage layer (112); and
b) a chip (120) comprising from bottom to top:
i) a culture medium chamber (121), having an inlet and an outlet allowing a culture medium to flow through the culture medium chamber;
ii) a membrane (122);
iii) a tissue chamber (123); and
iv) a cover (124);
the membrane (122) placed between the culture medium chamber (121) and the tissue chamber (123) separating said chambers, and the cover (124) sealing the tissue chamber (123);
wherein the osteochondral tissue (110) is placed in the tissue chamber having the subchondral bone layer (111) placed directly on top of the membrane and the cover placed on top of the cartilage layer (112).

2. The system of claim 1, wherein the subject from which the osteochondral tissue sample (110) has been extracted is a human or an animal subject, in particular a human subject, more in particular a healthy human subject or a human subject suffering from osteoarthritis.

3. The system of claim 1 or 2, wherein the osteochondral tissue sample (110) has been extracted from a joint of the subject, in particular from a synovial joint, and more in particular from a synovial joint selected from: compound joints, such as a knee joint; plane joints, such as a wrist carpal joint; hinge joints, such as an elbow joint; pivot joints such as an atlanto-axial joint, a proximal radioulnar joint, and a distal radioulnar joint; condyloid joints, such as a wrist radiocarpal joint; and ball and socket joints, such as a shoulder joint and a hip joint.

4. The system of any one of claims 1 to 3, wherein the osteochondral tissue sample (110) has the following dimensions:
- from 0.7 to 10 mm long, in particular from 1 to 8 mm and more in particular from 2 to 5 mm long
- from 0.7 to 10 mm wide, in particular from 1 to 8 mm and more in particular from 2 to 5 mm wide
- from 0.7 to 20 mm high, more particular from 1 to 15 mm and more in particular from 1 to 5 mm high.

5. The system of any one of claims 1 to 4, wherein the culture medium chamber (121) is connected to a pump.

6. The system of any one of claims 1 to 5, wherein the culture medium chamber (121) comprises a culture medium, preferably the culture medium comprises water, glucose and oxygen, and optionally further comprises additional components selected from amino acids, vitamins, inorganic salts, pyruvate, antibiotics and fetal bovine serum.

7. The system of any one of claims 1 to 6, wherein the membrane (122) is of a porous material, in particular a porous material having a pore size from 0.1 to 20 microns, more in particular from 0.2 to 15 microns, and yet more in particular from 0.4 to 10 microns.

8. The system of any one of claims 1 to 7, wherein the chip (120) comprises a material with low permeability to oxygen, in particular a polymeric material with low permeability to oxygen, and more in particular a polymeric material selected from a cyclic olefin polymer (COP), cyclic olefin copolymer (COC) and polyethylene terephthalate (PET).

9. A method for culturing an osteochondral tissue on a chip comprising culturing an osteochondral tissue sample (a) using a system (100) according to any one of claims 1 to 8, comprising allowing a culture medium to flow through the culture medium chamber (121), and preferably maintaining the system at a temperature from 35 to 39 °C, in particular from 36 to 38 °C.

10. The method of claim 9, comprising allowing a culture medium to flow through the culture medium chamber (121) at a flow rate from 0.1 to 10 µl/min, in particular from 0.5 to 5 µl/min, more in particular from 1 to 3 µl/min.

11. The method of claim 9 or 10, wherein culturing is performed from 5 to 90 days, in particular from 10 to 70 days, more in particular from 15 to 60 days yet more in particular from 20 to 50 days.

12. The method of any one of claims 9 to 11, wherein the culture medium is supplied as an incoming culture medium to the culture medium chamber (121) through the inlet of the culture medium chamber and the culture medium is collected as an outgoing culture medium through the outlet of the culture medium chamber, the culture medium providing nutrients from the incoming culture medium to the osteochondral tissue sample (110) through the membrane (122) and the culture medium collecting waste products from the osteochondral tissue sample (110) through the membrane (122) to provide the outgoing culture medium.

13. The method of any one of claims 9 to 12, comprising administering a pharmaceutical agent, a diagnostic agent and/or a metabolic agent to the osteochondral tissue sample (a), administering preferably comprising supplying the culture medium as an incoming culture medium according to the method of claim 12 wherein the incoming culture medium comprises the pharmaceutical agent the diagnostic agent and/or the metabolic agent.

14. The method of claim 13, further comprising monitoring the effect of the pharmaceutical agent, the diagnostic agent and/or the metabolic agent on the osteochondral tissue sample (110).

15. The method of claim 14, wherein monitoring comprises
- directly evaluating the osteochondral tissue sample (110), preferably using optical means; and/or
- indirectly evaluating the osteochondral tissue sample (110), preferably analyzing the composition of the culture medium, more preferably analyzing the composition of the outgoing culture medium provided according to claim 12.
